(19) **Europäisches Patentamt**

**European Patent Office**

**Office européen des brevets**

(11) **EP 0 824 019 B1**

(12) **EUROPEAN PATENT SPECIFICATION**

(45) Date of publication and mention
of the grant of the patent:
**20.11.2002 Bulletin 2002/47**

(51) Int Cl.7: **A61K 7/16**, A61K 7/28,
A61K 47/48

(21) Application number: **97305852.2**

(22) Date of filing: **01.08.1997**

(54) **Inhibition or reduction of oral malodour**

Hemmung oder Reduzierung des Mundgeruches

Suppression ou réduction de la forte haleine

(84) Designated Contracting States:
**CH DE FR GB LI NL**

(30) Priority: **13.08.1996 EP 96305925**

(43) Date of publication of application:
**18.02.1998 Bulletin 1998/08**

(73) Proprietor: **QUEST INTERNATIONAL B.V.**
**1411 GP Naarden (NL)**

(72) Inventors:
 • **Minhas, Tony**
   **Dartford, Kent DA1 2HZ (GB)**
 • **Casey, John**
   **Wellingborough,**
   **Northamptonshire NN9 6PL (GB)**
 • **Hyliands, Della**
   **Northampton NN5 5ET (GB)**

 • **James, Gordon**
   **Northamptonshire NN10 8LG (GB)**
 • **Mycock, Gary**
   **Northamptonshire NN10 8LH (GB)**
 • **Davis, Paul James**
   **Felmersham Bedfordshire, MK43 7EX (GB)**
 • **Beggs, Thomas Stewart**
   **Bedfordshire, MK44 2JX (GB)**

(74) Representative: **Matthews, Heather Clare et al**
   **Keith W Nash & Co**
   **Pearl Assurance House**
   **90-92 Regent Street**
   **Cambridge CB2 1DP (GB)**

(56) References cited:
   **EP-A- 0 140 498       EP-A- 0 380 084**
   **EP-A- 0 479 600       GB-A- 673 670**
   **GB-A- 2 047 091       US-A- 4 269 822**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the Invention

This invention relates to oral care, more specifically to the inhibition or reduction of oral malodour.

### Background to the Invention

The oral microflora is a complex ecosystem which contains a wide variety of microbial species. At least one oral species, the bacterium *Streptococcus mutans*, has the ability to utilise dietary carbohydrate for the synthesis of an insoluble polysaccharide matrix which facilitates attachment to and colonisation of hard surfaces.

Dental plaque consists of this matrix polysaccharide together with oral microorganisms which exist in and on the matrix polysaccharide.

The bacterial species *Streptococcus mutans* has been identified as an important, although not the sole, contributor to dental plaque and has been shown to be capable of inducing dental caries in germ-free animals when established as a mono-infection.

Other species which occur in dental plaque as a substantial proportion of the species normally present include *S. sanguis,* and *A. naeslundii.*

Dental plaque is an acidic micro environment. The acidity is generated by oral bacteria and their extracellular enzymes which convert dietary carbohydrate into the polysaccharide matrix. The acidity is damaging to the teeth bearing the oral bacteria and plaque, but can be tolerated by the bacteria themselves. The extracellular enzymes display greater activity under acidic conditions than in a neutral or alkaline environment.

There have been a number of proposals for delivering a biologically active agent, such as a cytotoxic enzyme, to a target site by means of an antibody having specific binding affinity for the target site.

The use of an antibody to attach a cytotoxic agent to a target site in the mouth has already been proposed. For example WO-A-89/11866 (Rama Biolink) discloses conjugates of bactericide and anti-*S.mutans.*

The delivery of enzymes as cytotoxic agents has been proposed. For example, Okuda et al, Infection and Immunity 27 690 (1980), describe use of two enzymes, xanthine oxidase and lactoperoxidase, chemically conjugated to antibodies against target cells. Over a period of ninety minutes, in vitro, they achieve a reduction of live candida albicans cells between one and two orders of magnitude better than that achieved with lactoperoxidase in solution.

Other disclosures of the use of antibodies or antibody fragments to target a cytotoxic agent to cells include WO90/0318 (Ideon) and WO91/00112 (Brunswick).

EP-A-450800, EP-A-451972, EP-A-453097 and EP-A-479600 (all Unilever) disclose the use of antibodies and antibody fragments to attach co-operating cytotoxic enzymes to a target site, especially oral bacteria.

### Summary of the Invention

According to the present invention there is provided a method of inhibiting or reducing oral malodour comprising applying topically in the mouth one or more vehicles which are acceptable for use therein, and which contain

(i) a polypeptide having specific binding affinity to a target site in the microflora on tooth surfaces,
(ii) an enzyme functional to generate an alkaline product and thereby raise pH,

said polypeptide and said enzyme being attached together or said vehicles or vehicles including means for attaching the enzyme to the polypeptide, at least at the time of use.

When performing the method of this invention, the enzyme is carried by the polypeptide and thereby bound to the target site which may be at or adjacent to dental plaque in the mouth. The enzyme functions to increase pH in the immediate locality. Such reduction of acidity through operation of the enzyme is directly beneficial to the teeth bearing the plaque because it is acidity which leads to degradation of tooth surfaces and the eventual formation of caries.

There is also a less direct benefit, in that the reduction in acidity also reduces the activity of the extracellular enzymes which are responsible for creating the acidity.

Furthermore, the creation of a less acidic microenvironment may reduce the competitive advantage of the acid-tolerant oral bacteria, and so favour colonisation of tooth surfaces by bacteria which are less harmful to the teeth.

An important factor in oral malodour, or bad breath, is $H_2S$, which is generated by organisms including *Streptococci,* such as *S. sanguis*. The volatility of $H_2S$ is pH dependent, with $H_2S$ dissociating to hydrogen ion and the soluble and highly reactive $HS^-$ ion with a $pK_a$ of about 7. At pH 8.0 over 90% of $H_2S$ is dissociated. The action in use of the enzyme in performing the method of the invention in raising pH therefore also promotes dissociation of $H_2S$ in the oral

cavity, so inhibiting or reducing oral malodour.

In another aspect, the invention covers use, as agents to inhibit or reduce oral malodour, of

(i) an enzyme functional to generate an alkaline product and thereby raise pH,
(ii) a polypeptide having specific binding affinity to a target site in the microflora on tooth surfaces, and serving also for attaching the enzyme, at least at the time of use.

It is envisaged that the enzyme will generally function to generate an alkaline product, and/or convert an alkaline substrate into a product which is more alkaline, thus increasing pH in its immediate locality. Alternatively, the enzyme may generate a precursor material which then undergoes a subsequent conversion *in situ* to an alkaline product. Such further conversion might be effected spontaneously by bacteria naturally present in plaque.

Enhancing the concentration of the precursor would then enhance the concentration of the alkaline product produced spontaneously from it.

The polypeptide with specific binding affinity to the target site may be an entire protein, or it may be a rather smaller polypeptide. It will usually be an antibody to the target site, or a fragment of an antibody which possesses the specific binding characteristics of an antibody.

Binding of a polypeptide to a target site entails ligand-receptor type interactions between the polypeptide and the material which is bound to it. There is an equilibrium between bound and unbound material, which can be represented by the chemical equation:

$$A+S \_ AS$$

where A represents unbound polypeptide, S represents unbound target sites and AS represents the complex of polypeptide and target bound together. The concentrations are related by:

$$\frac{[AS]}{[A][S]} = K_a$$

where $K_a$ is a constant, referred to as the affinity constant. Specific binding affinity between a polypeptide and a target will frequently have a value of $K_a$ of $10^6$ litre mole$^{-1}$ or greater. It may well have a value of at least $10^7$ or even at least $10^8$ litre mole$^{-1}$ for example in the range $10^7$ to $10^9$ litre mole$^{-1}$ which is typical of the binding affinity between an antibody and an antigen. However, even higher affinities, exceeding $10^9$ litre mole$^{-1}$ are possible and may be utilised within the scope of this invention.

This invention can be implemented using, as the binding polypeptide, polyclonal antibodies obtained by the traditional route of immunising a host animal with the target antigen (e.g. an oral bacterial species).

The invention can also utilise monoclonal antibodies, which can be derived by standard techniques from β-lyphocytes which make a polyclonal antibody response.

A further possibility for use as the binding polypeptide is an antibody fragment. Fragments which are used may be an Fab or Fv fragment, or the variable region of a heavy chain (a Dab fragment) or of a light chain. Some antibody fragments, notably Fab fragments, can be obtained by enzymic digestion of whole antibodies. Preferred routes to antibody fragments are through recombinant DNA technology, so that the fragment is expressed by a genetically transformed organism. These techniques may commence with DNA or mRNA from a hybridoma which produces monoclonal antibody, but other methods use immunoglobulin V-region gene libraries from immunised or non-immunised animals.

Antibody fragments produced by recombinant DNA technology need not be identical to fragments of antibodies produced in other ways. For instance they may include sequences of amino acids which differ from those found in antibodies produced in other ways, especially sequences at the ends of the fragments. Somewhat analogously, antibody fragments produced through recombinant DNA technology may include extra amino acid sequences at their termini which have no counterpart in antibodies produced in other ways.

Techniques for the production of antibody fragments are well known in the literature. Relevant disclosures include:

Saiki et al Science *230* 1350-54 (1985);
Orlandi et al PNAS USA *86* 3833-7 (1989);
WO 89/9825 (Celltech);
EP-A-368684 (Medical Research Council);
WO 91/8482 (Unilever).

Through use of recombinant DNA technology it is possible to produce polypeptides which incorporate fragments of more than one antibody, and consequently have specific binding affinity to more than one antigen. Such constructs may be used in the present invention.

A possibility is that a polypeptide binding agent for use in this invention is a synthetic polypeptide which mimics the specific binding activity of a natural antibody's complementarily determining region(s). Such a polypeptide is de *facto* a fragment of an artificial antibody.

An enzyme which is particularly envisaged for use in this invention is urease. This enzyme converts urea to ammonium hydroxide and carbon dioxide. The enzyme occurs in a number of bacterial, yeast and plant species. Urease from various sources is available from Sigma Chemical Company, PO Box 14508, St. Louis, MO 63178, USA and Sigma Chemical Ltd. Poole, Dorset, England. Species envisaged as sources of urease include *Bacillus pasteurii, Methophilus* and *Helicobacter pylori.*

Another enzyme which could be used is asparaginase, more completely designated as L-asparagine amidinohydrolase which converts L-asparagine into L-aspartic acid and ammonia, so that there is an overall increase in alkalinity. It has International Union of Biochemistry number 3.5.1.1 and is also available from Sigma.

Yet another possible enzyme is arginase, more specifically L-arginine amidinohydrolase, International Union of Biochemistry number 3.5.3.1. It is also available from Sigma and converts arginine to ornithine (which is more alkaline) and urea.

As mentioned above the enzyme may alternatively function to produce an intermediate which is not itself alkaline, but undergoes further reaction *in situ* to generate an alkaline product.

Binding of the enzyme to the polypeptide which serves to bind the enzyme to the target site may be accomplished in various ways. The binding may be direct conjugation by covalent chemical bonding to an antibody or antibody fragment which exhibits specific binding affinity for an oral microorganism.

Techniques for chemical conjugation of one polypeptide to another are known. Generally these use a pair of coupling agents which are reacted separately with respective polypeptides and then reacted with each other, linking the peptides together, when they are mixed.

An example of the conjugation of polypeptides is found in Knowles et al J. Clinical Investigation, *52* 1443, (1973) which used a modification of the technique of Dutton et al, Biochem Biophys. Res. Commun. *23* 730 (1966). Another example is provided in Lal et al, Journal of Immunological Methods *79* 307 (1985).

Chemical conjugation to an antibody fragment is likely to employ an Fab fragment or an Fv fragment which has been expressed with an additional "tail" as an extension of one of its peptide chains.

Another possibility is to conjugate the enzyme to an antibody or antibody fragment which does not bind directly to a species of the oral microflora, but instead binds to another antibody or fragment which will in turn bind to the target protein. This approach utilises chemical conjugation but does not require the antibody or fragment which binds the target protein to undergo a chemical reaction which might adversely affect it.

A further possibility is for the enzyme and an antibody fragment which binds to an oral microorganism to be formed as separate parts of a single polypeptide which is expressed as a fusion protein by a genetically transformed microorganism.

Alternatives to direct chemical bonding, relying on antibody-antigen binding, are also possible and a number of such techniques are described in our published European applications EP-A-450800, EP-A-453097, EP-A-451972 and EP-A-479600 mentioned above. In particular EP-A-453097 describes the linking of an antibody with specific binding affinity for a target site and an antibody with specific binding affinity for an enzyme by use of a third antibody which forms a bridge between them. A similar arrangement may also be achieved using antibody fragments as described in that European publication and also our EP-A-479600.

Another way to rely on an antibody-antigen type binding is to utilise a polypeptide which incorporates two antibody fragments as mentioned earlier. One of the fragments binds to the target site while the other binds to the enzyme. Suitable is that both fragments are Fv fragments.

WO 91/08482 referred to above describes the production of a polypeptide containing two $V_H$ chains with different specific binding affinities. WO 94/09131 describes the production of constructs containing two Fv antibody fragments with different specific binding affinities.

Holliger et al, Proc. Nat. Acad. Sci. USA *90* 6444 (1993) also described the production of polypeptides which each incorporate two Fv fragments.

In this invention, the target site may be one of the bacterial species which occur in dental plaque. The effect of targeting to one such species will be to raise pH both in the vicinity of organisms of that species and also in the surrounding areas of the plaque where other bacterial species may also occur. The matrix polysaccharide of dental plaque could itself be used as the target against which the antibody or antibody fragment displays specific binding affinity.

The invention uses one or more vehicles to contain the polypeptide (e.g. antibody or antibody fragment) with binding affinity to the target and also the enzyme whose function is to raise pH.

When the enzyme is directly attached to the binding polypeptide, as a fusion protein or conjugate, this can be contained in a single vehicle. However, if the enzyme is to be attached to an antibody or antibody fragment through antibody-antigen binding involving at least one further antibody or fragment, then it may well be desirable that the enzyme and antibodies or antibody fragments are distributed among a plurality of vehicles, so that the complex of enzyme and antibodies will form only when the vehicles are mixed, for example at the time of use. In this latter case, parts of the complex may link together beforehand if they are capable of doing so and are in the same vehicle but formation of the full complex will take place only when all of its components come together. Delaying full complex formation until the time of use can be advantageous in avoiding loss of activity during storage.

Complexes of enzyme and polypeptide which are large in size tend to be unstable, and to precipitate during storage. They also diffuse more slowly. For these reasons if whole antibodies are used it can be advantageous to distribute the enzyme and antibody(ies) between different vehicles, especially if the antibodies are polyclonal, since polyclonal antibodies tend to form larger complexes than monoclonal antibodies.

Antibody fragments have the advantage of being smaller, and form smaller complexes which would be expected to retain their activity during storage better than complexes formed with whole antibodies.

Plainly, for this invention, the vehicle or vehicles which are used should be suitable to enter the mouth. There are a number of possibilities.

The simplest possibility is a sterile aqueous solution for use as a mouthwash. This could be a one component mouthwash containing the binding polypeptide chemically conjugated to the enzyme. Alternatively, when the enzyme is not directly attached to the binding polypeptides, it could be a multicomponent mouthwash consisting of a plurality of solutions containing between them

(i) antibody or antibody fragment with specific binding affinity to a site in dental plaque,
(ii) the enzyme, e.g. urease,
(iii) antibody or antibody fragment with specific binding affinity to urease,
(iv) antibody or bivalent antibody fragment with specific binding affinity to the other antibodies or antibody fragments and which is able to a form bridge between them.

There could for example be one solution containing (i) and (iii) and a second solution containing (ii) and (iv).

These individual aqueous solutions or suspensions would be mixed together by the user before use as a mouthwash, allowing formation of a complex which in the case of whole antibodies would consist of anti-target antibody: bridging antibody: anti-enzyme antibody: enzyme, as described in our EP-A-453097.

Other possible forms of vehicle are a toothpaste, a lozenge which will dissolve in the mouth, chewing gum and dental floss. These forms of product could be used even when a plurality of vehicles are needed. A toothpaste with two components stored separately in a toothpaste container so that they are kept separate until the toothpaste is extruded from the container is a concept known *per se.* A lozenge to be sucked in the mouth could have various vehicles contained in separate regions of the lozenge so that the vehicles mix as the lozenge is sucked. Two different vehicle forms could be used in combination as a way to provide a plurality of vehicles: for example a toothpaste whose use is followed by a mouthwash or a lozenge.

The invention may use a product that includes a substrate for the enzyme or alternatively it may rely on the enzyme substrate being present at the target site.

For urease, the substrate is urea. Saliva contains a certain amount of urea, and we have found that this *in vivo* amount is a sufficiently high concentration to give changes of pH whose effect has been demonstrated *in vitro.*

A development of this invention is to use a product incorporating another enzyme with a beneficial effect which is also delivered by means of a polypeptide with specific binding affinity to a target site in the microflora on tooth surfaces, this further enzyme being suitable for giving its beneficial function in the alkaline environment created by the first enzyme. An enzyme which is particularly envisaged as the second enzyme is urate oxidase which functions to convert uric acid to carbon dioxide and allantoin at the same time generating hydrogen peroxide. Hydrogen peroxide is rapidly decomposed in vivo, but when the enzyme which generates it is located close to cells which it is desired to attack, then the hydrogen peroxide is able to give a cytotoxic action.

### Example 1

Monoclonal mouse antibodies with specific binding affinity to *S. sanguis* were produced by known techniques.

A urease-antibody conjugate was purchased from Sigma Chemical Ltd. It was urease chemically conjugated to a polyclonal goat anti-mouse antibody. The solution of this conjugate as supplied was diluted by varying amounts, as shown below, using sterile aqueous saline solution as diluent.

*S. sanguis* cells were suspended in sterile saline solution containing 5% w/v glucose and 0.1% w/v urea so as to provide a concentration of approximately $10^9$ cells ml$^{-1}$.

One sample of this suspension was used as a control. To another sample was added the urease conjugate at a dilution of 1/100.

To further samples were added the urease conjugate at the same or greater dilution, together with the mouse anti-*S. sanguis* antibody at a concentration of approximately 5μg/ml.

This acted as a bridging antibody which allowed the urease conjugate to bind to the *S. sanguis* cells. The complex which was formed consisted of the urease conjugate binding to the mouse antibody which in turn bound to the *S. sanguis* cells.

The pH of the medium was observed after periods of ½, 1, 2, 3 and 24 hours using a pH meter. The results are set out in Table 1.

Table 1

| | Observed pH of medium | | | | | |
|---|---|---|---|---|---|---|
| Elapsed Time (hrs) | 0 | 1/2 | 1 | 2 | 3 | 24 |
| Control (*S. sanguis* only) | 6.8 | 6.6 | 6.1 | 5.8 | 5.3 | 5.0 |
| *S. sanguis* + conjugate 1/100 dilution | 6.8 | 6.8 | 6.8 | 6.8 | 6.8 | 7.5 |
| *S . sanguis* + conjugate 1/100 dilution + mouse anti *S. sanguis* | 7.2 | 8.4 | 8.7 | 8.7 | 8.7 | 8.7 |
| *S . sanguis* + conjugate 1/500 dilution + mouse anti *S. sanguis* | 6.8 | 7.2 | 7.7 | 8.1 | 8.3 | 8.7 |
| *S . sanguis* + conjugate 1/1000 dilution + mouse anti *S. sanguis* | 6.8 | 6.9 | 7.1 | 7.3 | 7.4 | 7.5 |

As can be seen from these results, the *S. sanguis* on its own progressively made the medium acidic. Incorporation of the urease conjugate prevented this.

The pH remained stable over 3 hours and slowly thereafter became alkaline. Incorporation of the anti-*S. sanguis* antibody, so that the enzyme attached to the cells, led to alkaline pH much more quickly, even when the urease concentration was reduced.

*In vivo* in the mouth, it could be predicted from these results that urease conjugate which was not attached to cells or other target would be washed away by the saliva flow and have little effect, whereas urease which became attached to cells of the oral microflora would be retained in the mouth and be able to reduce or overcome the production of acidity.

## Example 2

Experiments were carried out *in vitro* to demonstrate the effect of treatment with urease and antibodies on $H_2S$ levels in the headspace above established *S. sanguis* biomass as a model of effects in the oral cavity of use of a method in accordance with the invention.

Below pH 4.0, $H_2S$ exists exclusively in the headspace. However, as the medium becomes more basic, $H_2S$ dissociates to the soluble and highly reactive $HS^-$ ion ($H_2S \rightleftarrows H^+ + HS^-$, $pK_a \sim 7$). In fact, at pH 8.0, > 90% of $H_2S$ is dissociated and dissolved in the medium. Above pH 10.0, $HS^-$ is further dissociated to $S^{2-}$. Significantly, in the presence of metal salts, $H_2S$ dissolution is essentially irreversible as the highly reactive $HS^-$ ion forms insoluble sulphide precipitates. In a preliminary study, the partial irreversible disappearance of $H_2S$ from the headspace in the presence of metal or ammonium salts was demonstrated experimentally (data not shown).

Established *S. sanguis* biomasses were resuspended in medium (amino acid-free except cysteine) as specified below, after being treated as outlined below.

The medium composition used for the growth of oral microorganisms was as follows:

**Basal medium (g/l):** $KNO_3$ (0.1); $KH_2PO_4$ (1.0); $K_2HPO_4$ (1.5); NaCl (0.1); $(NH_4)_2PO_4$ (2.0).

**Vitamins (mg/l):** nicotinic acid (1.0); nicotinamide (1.0); NADH (1.0); folic acid (1.0); calcium pantothenate (1.0); riboflavin (1.0); thiamine-HCl (1.0); spermine tetrahydrochloride (1.0); spermine trihydrochloride (1.0); putrescine dihydrochlaride (1.0); pimelic acid (0.1); D,L-mevalonic acid lactone (0.1); D-mevalonic acid lactone (0.1); biotin (0.1); *p*-aminobenzoic acid (0.1); lipoic acid (0.1); *B*-alanine (10.0); myo-inositol (10.0); choline chloride (50.0).

**Pyridoxal species (mg/l):** pyridoxal (1.0); pyridoxal-HCl (1.0); pyridoxamine dihydrochloride (1.0).

**Purines/pyrimidines (mg/l):** adenine (10.0); guanine (10.0); cytosine (10.0); thymine (10.0); xanthine (10.0); hypoxanthine (10.0); uracil (10.0).

**Salts (mg/l):** KI (4.0); $CuSO_4$ (1.0); $H_3BO_3$ (0.4); $FeSO_4$ (5.0); $MnSO_4$ (50.0); $Na_2MoO_4$ (5.0).

**Other additions:** hemin (5.0 mg/l); glucose (4.0g/l); $CaCl_2$ (10.0 mg/l); $MgSO_4$ (0.7g/l); $NaHCO_3$ (1.0g/l)

**Amino acids (mg/l):** cysteine (500).

The first antibody (1st Ab) employed in the study was a murine anti-*S. sanguis* IgG monoclonal used at a final concentration of 0.1 mg/ml (in blocking buffer). The first antibody binds to bacterial cells. The second antibody (2nd Ab) was a commerically available goat anti-mouse IgG preparation conjugated to Jack Bean urease (Sigma), which was diluted by a factor of 50 (in blocking buffer) prior to use. The second antibody binds to the first antibody and catalyses urea hydrolysis. The blocking buffer (TBS-Tween) contained 29 mM Tris-HCl pH 6.8, 0.15 M NaCl, 12.5 μM EDTA and 0.5% Tween 20. The blocking buffer prevents non-specific binding and is also employed to wash away unbound antibody.

Prior to resuspension in medium, each cell pellet was exposed to one of the following treatments (all at 30°C with agitation):

(i) untreated control;
(ii) 1 h incubation in TBS-Tween;
(iii) 1 h incubation in 1st Ab, followed by 2 x 15 min washes in TBS-Tween;
(iv) 1 h incubation in 2nd Ab, followed by 2 x 15 min washes in TBS-Tween;
(v) 1 h incubation in 1st Ab, 2 x 15 min washes in TBS-Tween, 1 h incubation in 2nd Ab, 2 x 15 min washes in TBS-Tween.

Headspace VSC levels were measured after 22 h and, after 23 h, the cells were harvested and resuspended in 10mM urea/12.5 μM EDTA (15 min incubation, with agitation, at 30°C). At this point, headspace VSC levels were again measured and the pH values of the suspensions recorded. Results are given in Table 2.

Table 2

| Treatment(s) | $H_2S$ @ t = 22h (ng/ml) | $H_2S$ @ t= 23.25h (ng/ml) | pH @ t = 23.25h |
|---|---|---|---|
| None | 218.6 | 120.7 | 5.4 |
| TBS-Tween | 357.4 | 160.3 | 5.3 |
| 1st Ab | 293.5 | 151.1 | 5.4 |
| 2nd Ab | 403.5 | 202.9 | 5.7 |
| 1st Ab + 2nd Ab | 272.9 | 2.3 | 8.5 |

The data presented in Table 2 clearly demonstrate that microbially-generated $H_2S$ is removed from the headspace due to a pH increase effected by the urease-catalysed production of ammonia from urea. The effect is clearly dependent on antibody targeting as no significant pH shift (or $H_2S$ removal) was observed when the urease-conjugated 2nd antibody was added on its own. Likewise, neither the 1st Ab nor the blocking buffer had any effect individually. Significantly, the effect is also long-lasting, an important prerequisite for use as an oral deodorant, as $H_2S$ removal was observed > 23 h after exposure of the cells to the antibodies.

A further requirement of active ingredients to control oral maladour is that they are capable of functioning after a short contact time. Consequently, a second experiment was undertaken to determine whether the antibody-targetted urease system could remove headspace $H_2S$ after only 5 min exposure to the S. sanguis cells.

For this experiment, established biomasses were resuspended as before in medium (amino acid-free except cysteine) after being treated as outlined below. The 1st and 2nd (urease-conjugated) Ab's were used at the same levels as outlined previously.

Prior to resuspension, treatments were as follows:

(i) 10 min incubation in TBS-Tween
(ii) 5 min incubation in 1st Ab, 5 min wash in TBS-Tween;
(iii) 5 min incubation in 2nd Ab, 5 min wash in TBS-Tween;
(iv) 5 min incubation in 1st Ab + 2nd Ab (mixture), 5 min wash in TBS-Tween.

Headspace VSC levels were measured after 18 h and, after 19 h, the cells were harvested and resuspended in 10 mM urea/12.5 μM EDTA (10 min incubation, with agitation, at 30°C). At this point, headspace VSC levels were again measured and the pH values of the suspensions recorded. After 20 h, the cells were pelleted and resuspended for a second time in urea/EDTA. On this occasion, the suspensions were degassed/overgassed with OFN an supplemented with chemically-generated $H_2S$ ($\sim$ 2000 ng/vial) before being incubated for 10 min as before. At this point, VSC and pH values were again recorded. Results are given in Table 3.

Table 3

| Treatment(s) | H$_2$S @ t=18h, ng/ml | H$_2$S @ t=19.2h, ng/ml (pH) | H$_2$S @ t=20.2h, ng/ml (pH) |
|---|---|---|---|
| TBS-Tween | 213.0 | 76.4 (pH 5.3) | 141.3 (pH 5.5) |
| 1st Ab | 226.0 | 86.5 (pH 5.3) | 137.9 (pH 5.4) |
| 2nd Ab | 207.4 | 70.7 (pH 8.3) | 131.7 (pH 5.9) |
| 1st Ab + 2nd Ab | 212.0 | 2.8 (pH 8.3) | 1.1 (pH 8.2) |

The data presented in Table 3 demonstrate that the antibody-targeted urease system is capable of removing headspace H$_2$S after only 5 min exposure to *S.sanguis* cells. Furthermore, the activity of the system was maintained after the addition of fresh medium and chemically-generated H$_2$S 20 h after initial exposure to the antibodies, confirming its continued effectiveness over long periods of time despite the diluting out of residual active material. The dependency of the system on antibody targeting was also confirmed.

The results of this study, as given in Tables 2 and 3, clearly demonstrate the potential of using antibody-targeted urease actives to prevent (or reverse) the volatility of microbially-produced H$_2$S in the oral cavity. Their viability as oral malodour counteractants is demonstrated by the fast, targeted, effective and long-lasting response they ellicit. *In situ,* it is envisaged that these actives may effect localised pH increases which could control volatile H$_2$S generation by *S.sanguis* and by other oral bacteria against which antibodies could also be raised.

**Claims**

1.  A method of inhibiting or reducing oral malodour comprising applying topically in the mouth one or more vehicles which are acceptable for use therein, and which contain

    (i) a polypeptide having specific binding affinity to a target site in the microflora on tooth surfaces,
    (ii) an enzyme functional to generate an alkaline product and thereby raise pH,

    said polypeptide and said enzyme being attached together or said vehicles or vehicles including means for attaching the enzyme to the polypeptide, at least at the time of use.

2.  A method according to claim 1, wherein the polypeptide has specific binding affinity to an oral bacterial species.

3.  A method according to claim 1 or 2, wherein the polypeptide is an antibody or an antibody fragment.

4.  A method according to claim 3, wherein the polypeptide incorporates fragments of two antibodies, one fragment having specific binding affinity to the said target site and the other fragment having specific binding affinity to the enzyme.

5.  A method according to claim 4, wherein the fragments are both Fv fragments.

6.  A method according to claim 3, wherein the said vehicle or vehicles include a second antibody or antibody fragment with specific binding affinity to the enzyme and a third antibody or antibody fragment able to bind to both the first said antibody or antibody fragment and to the second said antibody or antibody fragment.

7.  A method according to any one of claims 1 to 3, wherein the enzyme is directly attached to the polypeptide.

8.  A method according to claim 7, wherein the enzyme and the polypeptide with specific binding affinity are separate parts of a fusion protein.

9.  A method according to any one of claims 1 to 3, wherein the enzyme is directly attached to a second polypeptide

EP 0 824 019 B1

having specific binding affinity for the first said polypeptide.

10. A method according to any one of the preceding claims, wherein the vehicle or vehicles also incorporate an enzyme functional to generate hydrogen peroxide in an alkaline environment, said enzyme being attached to a polypeptide with specific binding affinity to a target site in the microflora on tooth surfaces or the product including means for attaching said second enzyme to such a polypeptide at least at the time of use.

11. Use, as agents to inhibit or reduce oral malodour, of

(i) an enzyme functional to generate an alkaline product and thereby raise pH,
(ii) a polypeptide having specific binding affinity to a target site in the microflora on tooth surfaces, and serving also for attaching the enzyme, at least at the time of use.

12. Use according to claim 11, wherein the polypeptide has specific binding affinity to an oral bacterial species.

13. Use according to claim 11 or 12, wherein the polypeptide is an antibody or an antibody fragment.

14. Use according to claim 13, wherein the polypeptide incorporates fragments of two antibodies, one fragment having specific binding affinity to the said target site and the other fragment having specific binding affinity to the enzyme.

15. Use according to claim 14, wherein the fragments are both Fv fragments.

16. Use according to claim 13, further comprising use of a second antibody or antibody fragment with specific binding affinity to the enzyme and a third antibody or antibody fragment able to bind to both the first said antibody or antibody fragment and to the second said antibody or antibody fragment.

17. Use according to any one of claims 11 to 13, wherein the enzyme is directly attached to the polypeptide.

18. Use according to claim 17, wherein the enzyme and the polypeptide with specific binding affinity are separate parts of a fusion protein.

19. Use according to any one of claims 11 to 13, wherein the enzyme is directly attached to a second polypeptide having specific binding affinity for the first said polypeptide.

20. Use according to any one claims 11 to 19, further comprising use of an enzyme functional to generate hydrogen peroxide in an alkaline environment, said enzyme being attached to a polypeptide with specific binding affinity to a target site in the microflora on tooth surfaces or the product including means-for attaching said second enzyme to such a polypeptide at least at the time of use.

**Patentansprüche**

1. Verfahren zur Inhibierung oder Reduktion von Mundgeruch, umfassend das topische Verabreichen im Mund von einem oder mehreren Vehikeln, die zur Verwendung darin akzeptabel sind und umfassen

(i) ein Polypeptid mit spezifischer Bindungsaffinität für einen Zielort in der Mikroflora auf Zahnoberflächen,
(ii) ein Enzym mit der Funktionalität zum Erzeugen eines alkalischen Produktes und dadurch zum Anheben des pH,

wobei das Polypeptid und das Enzym aneinander gebunden sind oder das Vehikel oder die Vehikel Möglichkeiten einschließen, das Enzym an das Polypeptid zu binden, zumindest zum Zeitpunkt der Verwendung.

2. Verfahren gemäß Anspruch 1, wobei das Polypeptid eine spezifische Bindungsaffinität bezüglich einer oralen bakteriellen Spezies aufweist.

3. Verfahren gemäß Anspruch 1 oder 2, wobei das Polypeptid ein Antikörper oder ein Antikörperfragment ist.

4. Verfahren gemäß Anspruch 3, wobei das Polypeptid Fragmente von zwei Antikörpern beinhaltet, wobei ein Fragment eine spezifische Bindungsaffinität in Bezug auf die Zielseite hat und das andere Fragment eine spezifische Bindungsaffinität in Bezug auf das Enzym hat.

5. Verfahren gemäß Anspruch 4, wobei die Fragmente beide Fv-Fragmente sind.

6. Verfahren gemäß Anspruch 3, wobei das Vehikel oder die Vehikel einen zweiten Antikörper oder ein zweites Antikörperfragment mit spezifischer Bindungsaffinität in Bezug auf das Enzym und einen dritten Antikörper oder ein drittes Antikörperfragment enthalten, der/das zur Bindung an sowohl den ersten Antikörper oder das erste Antikörperfragment als auch an den zweiten Antikörper oder das zweite Antikörperfragment fähig ist.

7. Verfahren gemäß mindestens einem der Ansprüche 1 bis 3, wobei das Enzym direkt an das Polypeptid gebunden ist.

8. Verfahren gemäß Anspruch 7, wobei das Enzym und das Polypeptid mit spezifischer Bindungsaffinität getrennte Teile eines Fusionsproteins sind.

9. Verfahren gemäß mindestens einem der Ansprüche 1 bis 3, wobei das Enzym direkt an ein zweites Polypeptid gebunden ist, das eine spezifische Bindungsaffinität für das erste Polypeptid aufweist.

10. Verfahren gemäß mindestens einem der vorherigen Ansprüche, wobei das Vehikel oder die Vehikel auch ein Enzym beinhalten, das zum Erzeugen von Wasserstoffperoxid in einer alkalischen Umgebung funktional ist, wobei das Enzym an das Polypeptid mit der spezifischen Bindungsaffinität in Bezug auf die Zielseite in der Mikroflora auf den Zahnoberflächen gebunden ist, oder das Produkt Möglichkeiten einschließt, das zweite Enzym an ein solches Polypeptid zumindest zum Zeitpunkt der Verwendung zu binden.

11. Verwendung als Mittel zum Inhibieren oder Reduzieren von Mundgeruch von

(i) einem Enzym, das zum Erzeugen eines alkalischen Produktes und dadurch zum Anheben des pH funktional ist,
(ii) einem Polypeptid mit spezifischer Bindungsaffinität an einen Zielort in der Mikroflora auf Zahnoberflächen, das auch zum Binden des Enzyms dient, zumindest zum Zeitpunkt der Verwendung.

12. Verwendung gemäß Anspruch 11, wobei das Polypeptid eine spezifische Bindungsaffinität an eine orale bakterielle Spezies aufweist.

13. Verwendung gemäß Anspruch 11 oder 12, wobei das Polypeptid ein Antikörper oder ein Antikörperfragment ist.

14. Verwendung gemäß Anspruch 13, wobei das Polypeptid Fragmente von zwei Antikörpern beinhaltet, wobei ein Fragment eine spezifische Bindungsaffinität in Bezug auf die Zielseite und das andere Fragment eine spezifische Bindungsaffinität in Bezug auf das Enzym aufweist.

15. Verwendung gemäß Anspruch 14, wobei die Fragmente beide Fv-Fragmente sind.

16. Verwendung gemäß Anspruch 13, die darüber hinaus die Verwendung eines zweiten Antikörpers oder eines zweiten Antikörperfragments mit spezifischer Bindungsaffinität in Bezug auf das Enzym und eines dritten Antikörper oder eines dritten Antikörperfragments umfasst, das zum Binden an sowohl den ersten Antikörper oder das erste Antikörperfragment als auch an den zweiten Antikörper oder das zweite Antikörperfragment fähig ist.

17. Verwendung gemäß mindestens einem der Ansprüche 11 bis 13, wobei das Enzym direkt an ein Polypeptid gebunden ist.

18. Verwendung gemäß Anspruch 17, wobei das Enzym und das Polypeptid mit spezifischer Bindungsaffinität separate Teile eines Fusionsproteins sind.

19. Verwendung gemäß mindestens einem der Ansprüche 11 bis 13, wobei das Enzym direkt an ein zweites Polypeptid mit spezifischer Bindungsaffinität für das erste Polypeptid gebunden ist.

20. Verwendung gemäß mindestens einem der Ansprüche 11 bis 19, die darüber hinaus die Verwendung eines Enzyms umfasst, das zum Erzeugen von Wasserstoffperoxid in einer alkalischen Umgebung funktional ist, wobei das Enzym an ein Polypeptid mit spezifischer Bindungsaffinität in Bezug auf eine Zielseite in der Mikroflora auf Zahnoberflächen gebunden ist, oder das Produkt Möglichkeiten zum Binden des zweiten Enzyms an ein solches Polypeptid zumindest zum Zeitpunkt der Verwendung beinhaltet.

**Revendications**

1.  Procédé pour inhiber ou réduire la mauvaise haleine, comprenant l'administration topique dans la bouche d'un ou plusieurs véhicules qui sont acceptables pour une utilisation dans celle-ci et qui comprennent

    (i) un polypeptide ayant une affinité de liaison spécifique pour un site cible dans la microflore sur les surfaces des dents,
    (ii) une enzyme fonctionnelle pour générer un produit alcalin et augmenter ainsi le pH,

    ledit polypeptide et ladite enzyme étant attachés ensemble ou auxdits véhicules ou à des véhicules comprenant un moyen pour fixer l'enzyme au polypeptide, au moins au moment de l'utilisation.

2.  Procédé selon la revendication 1, dans lequel le polypeptide a une affinité de liaison spécifique pour une espèce bactérienne orale.

3.  Procédé selon la revendication 1 ou 2, dans lequel le polypeptide est un anticorps ou un fragment d'anticorps.

4.  Procédé selon la revendication 3, dans lequel le polypeptide comprend des fragments de deux anticorps, un fragment ayant une affinité de liaison spécifique pour ledit site cible et l'autre fragment ayant une affinité de liaison spécifique pour l'enzyme.

5.  Procédé selon la revendication 4, dans lequel les fragments sont tous les deux des fragments Fv.

6.  Procédé selon la revendication 3, dans lequel ledit véhicule ou lesdits véhicules comprend/comprennent un second anticorps ou fragment d'anticorps avec une affinité de liaison spécifique pour l'enzyme et un troisième anticorps ou fragment d'anticorps capable de se lier à la fois audit premier anticorps ou fragment d'anticorps et audit second anticorps ou fragment d'anticorps.

7.  Procédé selon l'une quelconque des revendications 1 à 3, dans lequel l'enzyme est directement attachée au polypeptide.

8.  Procédé selon la revendication 7, dans lequel l'enzyme et le polypeptide avec une affinité de liaison spécifique sont des parties séparées d'une protéine de fusion.

9.  Procédé selon l'une quelconque des revendications 1 à 3, dans lequel l'enzyme est directement attachée à un second polypeptide ayant une affinité de liaison spécifique pour ledit premier polypeptide.

10. Procédé selon l'une quelconque des revendications précédentes, dans lequel le véhicule ou les véhicules comprend/comprennent aussi une enzyme fonctionnelle pour générer du peroxyde d'hydrogène dans un environnement alcalin, ladite enzyme étant attachée à un polypeptide avec une affinité de liaison spécifique pour un site cible dans la microflore sur les surfaces des dents ou le produit comprenant un moyen pour fixer ladite seconde enzyme à un tel polypeptide au moins au moment de l'utilisation.

11. Utilisation, comme agents pour inhiber ou réduire la mauvaise haleine, de

    (i) une enzyme fonctionnelle pour générer un produit alcalin et augmenter ainsi le pH,
    (ii) un polypeptide ayant une affinité de liaison spécifique pour un site cible dans la microflore sur les surfaces des dents, et servant aussi à fixer l'enzyme, au moins au moment de l'utilisation.

12. Utilisation selon la revendication 11, dans laquelle le polypeptide a une affinité de liaison spécifique pour une espèce bactérienne orale.

**13.** Utilisation selon la revendication 11 ou 12, dans laquelle le polypeptide est un anticorps ou un fragment d'anticorps.

**14.** Utilisation selon la revendication 13, dans laquelle le polypeptide comprend des fragments de deux anticorps, un fragment ayant une affinité de liaison spécifique pour ledit site cible et l'autre fragment ayant une affinité de liaison spécifique pour l'enzyme.

**15.** Utilisation selon la revendication 14, dans laquelle les fragments sont tous les deux des fragments Fv.

**16.** Utilisation selon la revendication 13, comprenant, en outre, l'utilisation d'un second anticorps ou fragment d'anticorps avec une affinité de liaison spécifique pour l'enzyme et d'un troisième anticorps ou fragment d'anticorps capable de se lier à la fois audit premier anticorps ou fragment d'anticorps et audit second anticorps ou fragment d'anticorps.

**17.** Utilisation selon l'une quelconque des revendications 11 à 13, dans laquelle l'enzyme est directement attachée au polypeptide.

**18.** Utilisation selon la revendication 17, dans laquelle l'enzyme et le polypeptide avec une affinité de liaison spécifique sont des parties séparées d'une protéine de fusion.

**19.** Utilisation selon l'une quelconque des revendications 11 à 13, dans laquelle l'enzyme est directement attachée à un second polypeptide ayant une affinité de liaison spécifique pour ledit premier polypeptide.

**20.** Utilisation selon l'une quelconque des revendications 11 à 19, comprenant, en outre, l'utilisation d'une enzyme fonctionnelle pour générer du peroxyde d'hydrogène dans un environnement alcalin, ladite enzyme étant attachée à un polypeptide avec une affinité de liaison spécifique pour un site cible dans la microflore sur les surfaces des dents ou le produit comprenant un moyen pour fixer ladite seconde enzyme à un tel polypeptide au moins au moment de l'utilisation.